(19) Europäisches Patentamt
European Patent Office
Office européen des brevets

(11) **EP 2 950 845 B1**

(12) **EUROPÄISCHE PATENTSCHRIFT**

(45) Veröffentlichungstag und Bekanntmachung des Hinweises auf die Patenterteilung:
**28.03.2018 Patentblatt 2018/13**

(21) Anmeldenummer: **14702785.8**

(22) Anmeldetag: **28.01.2014**

(51) Int Cl.:
**A61M 1/16** (2006.01)  **A61M 1/34** (2006.01)

(86) Internationale Anmeldenummer:
**PCT/EP2014/051615**

(87) Internationale Veröffentlichungsnummer:
**WO 2014/118168 (07.08.2014 Gazette 2014/32)**

(54) **VORRICHTUNG ZUR REGELUNG EINER BEHANDLUNGSVORRICHTUNG**

DEVICE FOR REGULATING A TREATMENT DEVICE

DISPOSITIF POUR REGULER UNE INSTALLATION DE TRAITEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.01.2013 DE 102013001587**
**30.01.2013 US 201361758485 P**

(43) Veröffentlichungstag der Anmeldung:
**09.12.2015 Patentblatt 2015/50**

(73) Patentinhaber: **Fresenius Medical Care Deutschland GmbH**
**61352 Bad Homburg (DE)**

(72) Erfinder:
• **HEIDE, Alexander**
**65817 Eppstein (DE)**
• **NIKOLIC, Dejan**
**65824 Schwalbach am Taunus (DE)**
• **PETERS, Arne**
**61352 Bad Homburg (DE)**
• **WIKTOR, Christoph**
**63571 Gelnhausen (DE)**

(74) Vertreter: **Dreyhsig, Jörg et al**
**Fresenius Medical Care AG & Co. KGaA**
**Global Intellectual Property**
**Frankfurter Strasse 6-8**
**66606 St. Wendel (DE)**

(56) Entgegenhaltungen:
EP-A2- 0 904 789      WO-A1-95/22743
WO-A1-99/67615      WO-A2-01/89599
WO-A2-2005/044339

**Beschreibung**

**Technisches Gebiet**

Die vorliegende Erfindung betrifft eine Vorrichtung zur Regelung einer Behandlungsvorrichtung, insbesondere zur Regelung der Ultrafiltration bei einer Dialysebehandlung.

**Hintergrund**

Die Dialyse ist ein Verfahren zur Blutreinigung von Patienten mit akuter oder chronischer Niereninsuffizienz. Grundsätzlich unterscheidet man hierbei Verfahren mit einem extrakorporalen Blutkreislauf, wie die Hämodialyse, die Hämofiltration oder die Hämodiafiltration und die Peritonealdialyse, die keinen extrakorporalen Blutkreislauf aufweist. Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, tritt Wasser aus dem Patientenblut über die Dialysemembran in den Dialysatkreislauf über, das sogenannte Ultrafiltrat. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator oder Hämofilter Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators oder Hämofilters zugesetzt wird oder stromabwärts, spricht man von Prä- oder Postdilution. Der Stoffaustausch erfolgt bei der Hämofiltration konvektiv.

Eine Kombination der Hämodialyse und der Hämofiltration liegt vor, wenn bei einer Dialysebehandlung dem Patientenblut gleichzeitig Substituat zugeführt wird. Diese Behandlungsform, die auch als Hämodiafiltration bezeichnet wird soll im Folgenden von dem Begriff der Hämodialyse, der Dialyse oder der Dialysebehandlung umfasst sein.

Bei der Dialysebehandlung ist es von entscheidender Bedeutung, dass der Flüssigkeitsentzug mit großer Genauigkeit gemessen und bilanziert wird, denn schon ein geringfügig zu großer Flüssigkeitsentzug könnte gravierende Folgen für den Patienten haben.

Dies wird dadurch sichergestellt, dass der Zufluss des Dialysats oder der Dialysierflüssigkeit in die Dialysierflüssigkeitskammer und der Abfluss der Dialysierflüssigkeit aus der Dialysierflüssigkeitskammer getrennt von einander kontrolliert werden. Die Bilanz zwischen der der Dialysierflüssigkeitskammer zugeführten Flüssigkeitsmenge und der aus der Dialysierflüssigkeitskammer abgeführten Flüssigkeitsmenge gibt dabei gleichzeitig ein Maß des dem Patientenblut entzogenen Ultrafiltrats an.

Eine Möglichkeit zur Bilanzierung ist der Einsatz von Bilanzkammerpumpen, die auf dem Prinzip beruhen, dass eine zugeführte Flüssigkeitsmenge in einem Zufluss zur Dialyiserflüssigkeitskammer einer in einen Abfluss aus der Dialysierflüssigkeitskammer abgeführten Flüssigkeitsmenge entspricht.

Für das zusätzliche Entziehen von Flüssigkeit aus dem Patienten wird ein weiterer Flussweg mit Fördereinrichtung, die sog. Ultrafiltrationspumpe, parallel zur Bilanzkammer angeordnet. Die abzuziehende Flüssigkeit wird dabei über den parallelen Flussweg an der Bilanzkammer vorbei und durch die Ultrafiltrationspumpe gemessen und bildet so ein Maß für die Flüssigkeitsbilanz.

Bilanzkammern sind konstruktiv aufwendig und stellen hohe Anforderungen an die Fertigungstoleranz.

Alternativ kann die Kontrolle der Ultrafiltration, durch die Kontrolle der Flussrate in der Zuleitung zur Dialysierflüssigkeitskammer und der Flussrate in der Ableitung aus der Dialysierflüssigkeitskammer durch unabhängig voneinander ansteuerbare jeweils in der Zuleitung und in der Ableitung angeordnete Pumpen erfolgen. In diesem Fall erfolgt die Bilanzierung durch jeweilige in der Zu- und in der Ableitung angeordnete Flusssensoren oder Waagen, was mit einem hohen Aufwand für die Kalibrierung dieser Sensoren oder Waagen verbunden ist.

In diesem Zusammenhang ist die WO2005/044339 zu nennen, die verschiedene Vorrichtungen und Methoden für Heim Hämodialysebehandlungen offenbart die den Umfang der Rückfiltrierung ("Backfiltration") erhöhen. Das System verwendet ein wegwerfbares Fluidmanagementsystem, dass ein Wegwerfset mit einer wegwerfbaren Kassette oder einer Schlauchsatzhalterung umfassen kann. Die Kassette umfasst mindestens einen Teil der Dialysat- und der extrakorporalen Flusswege. In einer Ausführungsform sind zwei Hochfluss-Dialysatoren flüssigkeitsmäßig und in Serie mit der Kassette verbunden.

Eine Drosselvorrichtung ist zwischen den Dialysatoren im Dialysierflüssigkeitspfad angeordnet. In einer Ausführungsform ist die Drosselvorrichtung variabel und einstellbar, um verschiedenen Behandlungssituationen Rechnung zu tragen oder um während einer einzelnen Behandlung angepasst zu werden. In einer anderen Ausführungsform ist die Drosselvorrichtung fest vorgegeben. Aufgrund der Drosselung zwischen den Dialysatoren wird ein positiver Druck in dem venösen Dialysator (dem ersten Dialysator, in den Dialysat strömt, aber der zweite Dialysator, in den Blut strömt in einer Gegenstromanordnung) erzeugt, wodurch ein relativ großes Maß an Rückflitration bewirkt wird. In Abhängigkeit von dem Ausmaß der Drosselung zwischen den Dialysatoren, bewirkt diese Rückfiltration einen signifikanten Fluss (z.B. 10 bis 70 Prozent des gesamten Dialysatflusses) von Dialysat durch die venösen Hochflussmembranen in die Blutleitung. Die rückgefilterte Lösung bewirkt eine konvektive Clearance.

Zu der Steuerung der Flussverhältnisse am Dialysator wird folgendes offenbart:

Das [Dialyse-]System setzt Pumpen ein, die mit entsprechenden Vorrichtungen zur Volumenmessung zusammenwirken. Eine Kontrollvorrichtung oder Mikroprozessor vergleicht die tatsächliche Flüssigkeitsmenge mit einer erwarteten Flüssigkeitsmenge die gepumpt wurde und passt Pumpraten entsprechend an, um die Förderung von Flüssigkeit zu den Dialysatoren nach Bedarf zu beschleunigen oder zurückzuhalten..

Auch die WO 95/22743 offenbart eine Dialysemaschine, bei der Strömungsregler stromauf und stromab eines Dialysators, aus Sicht der Fließrichtung der Dialysierflüssigkeit, angeordnet sind. Jeder Strömungsregler weist jeweils einen Flussbegrenzer, einen Druckmesser, sowie eine Pumpe auf. Zusätzlich ist ein Flussmessgerät zwischen den Strömungsreglern angeordnet, um die Flüsse zu und von dem Dialysator zu messen. Das Flussmessgerät weist zwei Messzellen auf. Eine der Messzellen ist jeweils ausgeführt, um den vorgegebenen Wert des entsprechenden Drucksensors des Konstantströmungsreglers zu steuern. Ein Mikroprozessor regelt die Pumpen der Strömungsregler so, dass der Druck der jeweiligen Drucksensoren einem entsprechenden vorgegebenen Wert entspricht. Gemäß der Erfindung der WO 95/22743 werden die Signale der Drucksensoren zusätzlich zur Berechnung des Flusses durch die Drosselvorrichtungen oder Flussbegrenzer anhand einer bestimmten Formel verwendet. Eine Möglichkeit der Einstellung einer der Flussbegrenzer ist gemäß der Offenbarung der WO 95/22743 nicht gegeben.

Es ist daher eine Aufgabe der Erfindung, zumindest eine der oben genannten Schwierigkeiten zu überwinden und eine einfache Vorrichtung zur Regelung der Ultrafiltration zur Verfügung zu stellen.

## Zusammenfassung

Die Erfindung wird durch die Merkmale der unabhängigen Ansprüche 1 und 3 definiert.

Diese Aufgabe wird gelöst durch eine Vorrichtung zur Regelung einer Ultrafiltration bei einer Dialysebehandlung bei der zu ultrafiltrierendes Blut in einem extrakorporalen Blutkreislauf eine Blutkammer eines durch eine semipermeable Membran in die Blutkammer und eine Dialysierflüssigkeitskammer unterteilten Dialysators durchströmt und Dialysierflüssigkeit in einem Dialysierflüssigkeitskreislauf die Dialysierflüssigkeitskammer des Dialysators durchströmt. Die offenbarungsgemäße Vorrichtung weist eine Blutpumpe zur Kontrolle eines Blutflusses in dem extrakorporalen Blutkreislauf, eine in dem Dialyssierflüssigkeitskreislauf in ein einem Zustrom zu dem Dialysator angeordneten Dialysierflüssigkeitspumpe zur Kontrolle eines Dialysierflüssigkeitsflusses in dem Zustrom, eine in dem Dialysierflüssigkeitskreislauf in einem Abfluss aus dem Dialysator angeordnete Drossel zur Kontrolle des Dialysierflüssigkeitsflusses in dem Abfluss eine Bilanziervorrichtung zum Aufstellen einer Flüssigkeitsbilanz im Dialysierflüssigkeitskreislauf zwischen dem Zustrom zu und dem Abfluss aus der Dialysierflüssigkeitskammer als Maß für die Ultrafiltration, sowie einer Regeleinheit zum Regeln der Blutpumpe, der Dialysierflüssigkeitspumpe und/oder der Drossel auf. Die Regelung der Drossel erfolgt so, dass eine vorbestimmte Ultrafiltration erzielt wird.

In einer alternativen Ausführungsform ist die Drossel stromauf des Dialysators in dem Zufluss zur Dialysierflüssigkeitskammer und die Dialysierflüssigkeitspumpe stromab des Dialysators in dem Abfluss aus der Dialysierflüssigkeitskammer angeordnet.

Weiterhin wird die vorliegende Aufgabe durch eine Vorrichtung nach Anspruch 1 oder 3 gelöst. Vorteilhafte Ausführungsformen sind in den abhängigen Ansprüchen angegeben.

Die Anordnung der Dialysierflüssigkeitspumpe im Dialysierflüssigkeitskreislauf in einem Zufluss zu der Dialysierflüssigkeitskammer bedingt, dass die Dialysierflüssigkeitspumpe nahe an einer Diaylsierflüssigkeitszubereitung angeordnet ist.

Die Erfinder haben erkannt, dass bei dieser Konfiguration zur Regelung des Dialysierflüssigkeitsflusses keine weitere Dialysierflüssigkeitspumpe stromab der Dialysierflüssigkeitskammer erforderlich ist. Dies geht mit einem verminderten konstruktiven Aufwand einher. Entsprechendes gilt, wenn die Dialysierflüssigkeitspumpe in dem Abfluss aus der Dialysierflüssigkeitskammer angeordnet ist. In dieser Konfiguration ist keine weitere Dialysierflüssigkeitspumpe zur Regelung des Dialysierflüssigkeitsflusses stromauf der Dialysierflüssigkeitskammer erforderlich.

Vorteilhaft ist die Blutpumpe im Blutkreislauf in einer Zuleitung zu der Blutkammer angeordnet. Auf diese Weise trägt der durch die Blutpumpe bereitgestellte Druck zu einem Überdruck in der Blutkammer gegenüber der Dialysierflüssigkeitskammer bei.

Die Regelung der Ultrafiltration kann so erfolgen, dass für die Blutflussrate oder für den von der Blutpumpe aufgebrachten Druck ein vorbestimmter Wert einstellbar ist und die Regeleinheit die Ultrafiltration dadurch regelt, dass die Dialysierflüssigkeitspumpe und/oder die Drossel in Abhängigkeit von einer gemessenen Ultrafiltrationsrate gesteuert geregelt wird, indem ein Druck oder ein Volumenstrom in einer Zuleitung zu der Dialysierflüssigkeitskammer im Dialysierflüssigkeitskreislauf sowie der Drosselwiderstand entsprechend geregelt werden.

In einem nicht zur Erfindung gehörenden Beispiel kann die Regelung der Ultrafiltration auch so erfolgen, dass für die Dialysierflüssigkeitsrate oder einen Förderdruck der Dialysierflüssigkeitspumpe sowie für den Drosselwert vorbestimmte Werte einstellbar sind und die Regeleinheit die Ultrafiltration dadurch regelt, dass sie die Blutpumpe in Abhängigkeit von einer gemessenen Ultrafiltrationsrate regelt, indem der Druck oder der Volumenstrom in einer Zuleitung zu der Blutkammer im extrakorporalen Blutkreislauf entsprechend geregelt wird.

Es ist aber jede andere Regelung der Ultrafiltration möglich, so lange durch die Blutpumpe im Blutkreislauf, sowie durch die Dialysierflüssigkeitspumpe und die Drossel im Dialysierflüssigkeitskreislauf die Druckverhältnisse am Dialysator so gesteuert und/oder geregelt werden können, dass eine gewünschte Ultrafiltration stattfindet.

Die Drossel kann als Drossel mit einem variabel einstellbaren Querschnitt als Durchfluss, als getaktetes Ventil oder als anderes Bauelement ausgeführt sein, dessen Fluidwiderstand variabel einstellbar ist, einschließlich eines variabel einstellbaren Fluidwiderstandes, der sich erst durch eine zeitliche Mittelung ergibt.

In einer Weiterbildung der Vorrichtung weist die Bilanziervorrichtung eine Differenzflussmesseinheit zum Messen des Differenzflusses zwischen einem Fluss in dem Zustrom zur Dialysierflüssigkeitskammer und dem Abfluss aus der Dialysierflüssigkeitskammer, eine Verzweigung von dem Zufluss oder dem Abfluss zur Abzweigung von Dialysierflüssigkeit von dem Zufluss oder dem Abfluss in einen weiteren Flussweg, sowie eine Einrichtung zur Einstellung der Flussmenge in dem Zufluss, in dem Abfluss und/ oder in dem weiteren Flussweg, auf, die derart ansteuerbar ist, dass der gemessene Differenzfluss eine vorbestimmte Bedingung erfüllt. In dieser Weiterbildung weist die Vorrichtung weiterhin eine Einrichtung zur Ermittlung der Flussmenge in dem weiteren Flussweg als Maß der Flüssigkeitsbilanz auf.

Der Fluss in dem weiteren Flussweg kann etwa mit einer in dem Flussweg angeordneten Pumpe oder Drossel eingestellt werden.

## Kurzbeschreibung der Figuren

Fig. 1 zeigt ein Blockdiagramm eines Dialysegerätes mit einer Vorrichtung zur Regelung der Ultrafiltration.

Fig. 2 zeigt ein Blockdiagramm eines weiteren Dialysegeräts mit einer weiteren Vorrichtung zur Regelung der Ultrafiltration.

Fig. 3 zeigt ein Blockdiagramm eines Ersatzschaltbildes für eine Dialysevorrichtung.

Fig. 4 zeigt ein Blockdiagramm eines vereinfachten elektrischen Ersatzschaltbildes für eine Dialysevorrichtung.

Fig. 5 zeigt ein Blockdiagramm eines Hämofiltrationsgerätes mit einer Vorrichtung zur Regelung der Hämofiltration.

## Detaillierte Beschreibung der Figuren

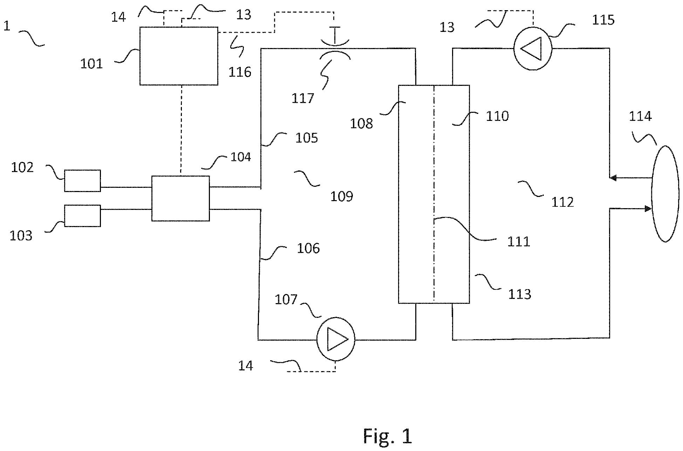

In Figur 1 ist schematisch ein Dialysegerät 1 mit einer Vorrichtung zur Regelung der Ultrafiltration im Einklang mit der Lehre der vorliegenden Erfindung dargestellt. Das zu behandelnde Blut wird über einen Zugang 114 dem Patienten entnommen und mit einer Blutpumpe 115 im extrakorporalen Blutkreislauf 112 durch eine Blutkammer des Dialysators 113 und über den Zugang 114 an den Patienten zurückgegeben. Der Zugang 114 verbindet den Blutkreislauf 112 mit einem Blutgefäß des Patienten, das zur Blutentnahme und Rückgabe geeignet ist. Der Zugang 114 kann zur Blutentnahme und zur Rückgabe des Blutes einen getrennten Ab- und Zufluss enthalten ('Double Needle'- Verfahren), oder Zu- und Abfluss können als ein Element ausgeführt sein ('Single Needle'- Verfahren).

Im Dialysator 113 trennt eine semipermeable Membran 111 eine Dialysierflüssigkeitskammer 108 von einer Blutkammer 110. Über die semipermeable Membran 111 findet ein Flüssigkeits- und Stoffaustausch von der Blutkammer 110 in die Dialysierflüssigkeitskammer 108 statt. Durch die Dialysierflüssigkeitskammer 108 des Filters 113 wird Dialysierflüssigkeit im Dialysierflüssigkeitskreislauf 109 mit einer Dialysierflüssigkeitspumpe 107 in einer Zuleitung 106 stromauf der Dialysierflüssigkeitskammer transportiert. Außerdem wird der Dialysierflüssigkeitsfluss in der Ableitung 105 aus der Dialysierflüssigkeitskammer 108 mittels der variabel einstellbaren Drossel 117 kontrolliert. Alternativ kann die Dialysierflüssigkeitspumpe 107 auch in der Ableitung 105 stromab der Dialysierflüssigkeitskammer 108 angeordnet sein und die Kontrolle des Dialysierflüsskeitsflusses in der Zuleitung 106 erfolgt durch eine variabel einstellbare Drossel 107. Im von einer Dialysierflüssigkeitsquelle 103 gespeisten Dialysierflüssigkeitskreislauf 109 ist eine Bilanziervorrichtung 104 angeordnet, zur Bilanzierung der der Dialysierflüssigkeitskammer 108 zugeführten und der aus dem Dialysator 113 abfließenden Dialysierflüssigkeit. Hierzu kann die Flussrate im Zufluss zur Dialysierflüssigkeitskammer 108 und die Flussrate im Abfluss der Dialysierflüssigkeitskammer 108 separat erfasst werden, oder es kann ein Differenzfluss als Maß für die Flüssigkeitsbilanz ermittelt werden. Die Flüssigkeitsbilanz entspricht der über die Membran 111 im Dialysator 113 entzogenen Ultrafiltrationsmenge. Aus dem Dialysator 113 abfließende, sogenannte verbrauchte Dialysierflüssigkeit wird im Allgemeinen in einen Dialysierflüssigkeitsabfluss 102 verworfen. Alternativ kann eine Regeneration verbrauchter Dialysierflüssigkeit vorgesehen sein.

Zusätzlich kann im Fall der Hämodiafiltration stromauf oder stromab des Dialysators 113 im Blutkreislauf 512 eine Zugabe von Substitutionsflüssigkeit durch eine Substituatleitung (nicht gezeigt) erfolgen. In diesem Fall ist die zugefügte Substituatmenge bei der Gesamtflüssigkeitsbilanz mit zu berücksichtigen.

Durch die Steuerung der Blutpumpe 115, die Steuerung der Dialysierflüssigkeitspumpe 107, und/oder die Steuerung der variabel einstellbaren Drossel 117 werden die Druckverhältnisse an der Membran 111 im Dialysator 113 so beeinflusst, dass in der Blutkammer 110 gegenüber der Dialysierflüssigkeitskammer 108 ein Überdruck herrscht. Dadurch erfolgt ein Transport von Flüssigkeit durch die Membran von der Blutkammer 110 in die Dialysierflüssigkeitskammer 108.

Die Blutpumpe 115 kann zum Erzielen einer bestimmten Pumpendrehzahl oder einer bestimmten Blutflussrate als Betriebsparameter ansteuerbar sein, etwa in einer Ausführung als peristaltische Pumpe. Alternativ kann die Blutpumpe 115 zum Erzielen eines bestimmten Förderdrucks als Betriebsparameter ansteuerbar sein, etwa als Impellerpumpe.

Ebenso kann die Dialysierflüssigkeitspumpe 107 zum Erzielen einer bestimmten Förderrate oder Pumpendrehzahl als peristaltische Pumpe, als Membranpumpe, als Kolbenpumpe o. Ä. ausgeführt sein oder zum Aufbauen eines bestimmten Förderdrucks, z.B. als Impellerpumpe.

Die variable einstellbare Drossel 117 kann als Drossel mit einem variabel einstellbaren Querschnitt als Durchfluss, als getaktetes Ventil oder als anderes Bauelement ausgeführt sein, dessen Fluidwiderstand variabel einstellbar ist, einschließlich eines variabel einstellbaren Fluidwiderstandes, der sich erst durch eine zeitliche Mittelung ergibt.

Eine mit der Bilanziervorrichtung 104 über eine Messleitung verbundene Steuer- und Regeleinheit 101 ist über die Steuerleitung 13 mit der Blutpumpe 115 über die Steuerleitung 14 mit der Dialysierflüssigkeitspumpe 107, sowie über die Steuerleitung 116 mit der variabel einstellbaren Drossel 117 verbunden. Während der Blutbehandlung werden kontinuierlich oder periodisch aktuelle Messparameter der Ultrafiltration, etwa der Ultrafiltrationsmenge oder Ultrafiltrationsrate von der Bilanziervorrichtung an die Steuer- und Regeleinheit 101 übermittelt. Die Steuer- und Regeleinheit 101 verwendet die aktuellen Messparameter um Steuersignale für die die Blutpumpe 115, die Dialysierflüssigkeitspumpe 107, sowie für die variabel einstellbare Drossel 117 abzuleiten. Die Ansteuerung der Dialysierflüssigkeitspumpe 107, der Blutpumpe 115, und der variabel einstellbaren Drossel 117 erfolgt dabei im Hinblick auf eine zu erzielende Ultrafiltration, etwa eine bestimmte Ultrafiltrationsrate oder ein bestimmtes, über den Behandlungsverlauf zu erzielendes Ultrafiltrationsvolumen.

Die Regelung kann etwa so erfolgen, dass die Blutpumpe 115 sowie die Dialysierflüssigkeitspumpe 107 mit einer konstanten Drehzahl oder mit einem konstanten Förderdruck betrieben wird, und die variabel einstellbare Drossel 117 so gesteuert wird, dass der von der Bilanziervorrichtung 104 übermittelte Ultrafiltrationswert als Regelgröße dient. Liegt etwa der übermittelte gemessene Wert der Ultrafiltrationsrate über einem entsprechenden Sollwert, so ist die variabel einstellbare Drossel 117 zu verengen, oder mit anderen Worten: ihr Flusswiderstand ist zu erhöhen, liegt die Ultrafiltrationsrate unter ihrem Sollwert, wird die variabel einstellbare Drossel 117 geweitet, oder mit anderen Worten: ihr Flusswiderstand wird vermindert.

In einem nicht zur Erfindung gehörenden Beispiel ist die Regelstrategie derart, dass die Blutpumpe 115 mit einer konstanten Drehzahl oder mit einem konstanten Förderdruck betrieben wird, die variabel einstellbare Drossel 117 auf einen bestimmten Flusswiderstand eingestellt wird und die Dialysierflüssigkeitspumpe 107 so gesteuert wird, dass der von der Bilanziervorrichtung 104 übermittelte Ultrafiltrationswert als Regelgröße dient. Liegt etwa der übermittelte Wert der Ultrafiltrationsrate über einem entsprechenden Sollwert, so ist die Dialysierflüssigkeitspumpe 107 zu beschleunigen, liegt die Ultrafiltrationsrate unter ihrem Sollwert, wird die Dialysierflüssigkeitspumpe 107 gedrosselt.

Eine weitere nicht zur Erfindung gehörende Regelstrategie kann sein, die Dialysierflüssigkeitspumpe 107 mit einer konstanten Drehzahl oder mit einem konstanten Förderdruck zu betreiben, die variabel einstellbare Drossel 117 auf einen bestimmten Flusswiderstand einzustellen und die Blutpumpe 115 so anzusteuern, dass der von der Bilanziervorrichtung 104 übermittelte Ultrafiltrationswert geregelt wird. Liegt etwa die übermittelte der Wert der Ultrafiltrationsrate über einem entsprechenden Sollwert, so ist die Blutpumpe 115 zu drosseln, liegt die Ultrafiltrationsrate unter ihrem Sollwert, so wird die Blutpumpe 115 beschleunigt.

Eine Kombination der Regelstrategien ist möglich, etwa in der Art, dass in einer inneren Regelschleife zunächst die Blutpumpe 115 und/oder die Dialysierflüssigkeitspumpe 107 konstant betrieben werden wird und die variabel einstellbare Drossel 117 gesteuert wird. Erst wenn die Drossel vollständig geöffnet wird, werden die Blutpumpe 115 und/oder die Dialysierflüssigkeitspumpe 107 entsprechend angesteuert.

Die Regelung der Ultrafiltration kann so erfolgen, dass für die Ultrafiltrationsrate ein bestimmter Wert vorgegeben wird. Alternativ kann für das während der Blutbehandlung zu entziehende Ultrafiltrationsvolumen ein bestimmtes Ultrafiltrationsprofil vorgegeben werden.

Der vorgegebene Wert der Ultrafiltrationsrate kann ein konstanter oder sich kontinuierlich ändernder Wert für die Ultrafiltrationsrate sein.

Alternativ kann für die Ultrafiltrationsrate oder das Ultrafiltrationsvolumen ein Profil vorgegeben werden, bei dem sich Intervalle mit einer positiven Ultrafiltrationsrate und Intervalle mit einer negativen Ultrafiltrationsrate abwechseln. Auf diese Weise kann ein sogenannter push/pull Modus erreicht werden, bei dem Ablagerungen von der Dialysatormembran abgelöst oder ein Anlagern von Substanzen an die Dialysatormembran vermindert oder verhindert wird. Dadurch wird die Durchlässigkeit der Dialysatormembran sowie die entsprechende Reinigungsleistung (Clearance) für Mittelmoleküle verbessert. Die vorliegende Anordnung erreicht dies mit geringem apparativen Aufwand, etwa: ohne eine zusätzliche Pumpe für das Aufbringen oszillierender Druckpulse. So kann ein push/pull Modus ausschließlich durch entsprechendes getaktetes Ansteuern der variabel einstellbaren Drossel 117 erreicht werden.

Die Regelung der Ultrafiltration kann in diesem Fall analog zu den oben für die Ultrafiltrationsrate beschriebenen Regelstrategien erfolgen, wobei an die Stelle eines Abgleichs mit dem Sollwert der Ultrafiltrationsrate ein entsprechender Abgleich mit einem Ultrafiltrationsprofil erfolgt.

Im Falle der Hamodiafiltration kann die während der Behandlung über die Substituatleitung zugefügte Substituatmenge in die Flüssigkeitsbilanz eingestellt werden und die resultierende Gesamtultrafiltrationsrate, d.h. die Differenz der über den Dialystor entzogenen Flüssigkeitsmenge und der Substituatrate wird als Regelgröße herangezogen.

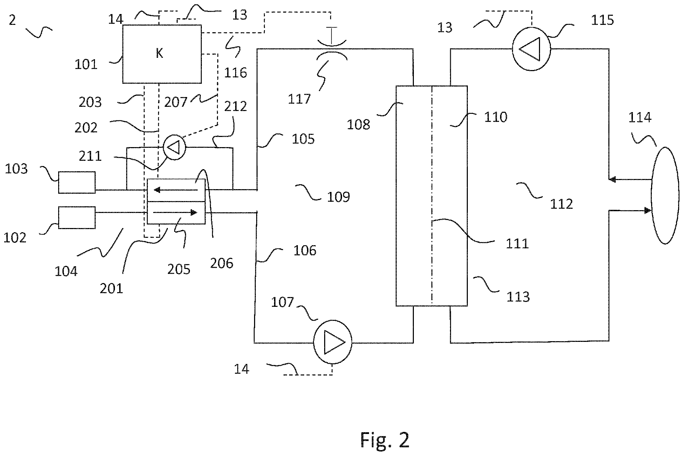

Figur 2 zeigt schematisch ein weiteres Dialysegerät mit einer weiteren Vorrichtung zur Regelung der Ultrafiltration. Das in Figur 2 dargestellte Dialysegerät entspricht im Wesentlichen dem Aufbau des Ultrafiltrationsgeräts der Figur 1. Auf die Beschreibung der entsprechenden Elemente wird Bezug genommen an Stelle einer Wiederholung. Die Darstellung des Ultrafiltrationsgeräts unterscheidet sich im Wesentlichen durch die Ausführung der Bilanziervorrichtung 104, die im Folgenden genauer beschrieben wird.

Die Bilanziervorrichtung 104 umfasst die zu einem Differenzstromsensor 201 verbundenen Flussmesszellen 205 und 206, wobei die Flussmesszelle 205 stromauf der Dialysierflüssigkeitskammer 108 und die Flussmesszelle 206 stromab der Dialysierflüssigkeitskammer 108 im Dialysierflüssigkeitskreislauf 109 liegt.

Eine Ultrafiltrationspumpe 211 liegt in einem zur Flussmesszelle 206 parallelen Flüssigkeitsweg 212, in dem der Flüssigkeitstransport durch die Ultrafiltrationspumpe 211 kontrolliert wird.

An Stelle der Ultrafiltrationspumpe 211 kann auch eine Drossel vorgesehen sein, um den Fluss in dem parallelen Flüssigkeitsweg 212 zu kontrollieren.

Der Differenzstromsensor 201 ermittelt ein Messwertpaar bestehend aus einem separaten Messwert für jede Flussmesszelle 205, 206, der die Strömungsgeschwindigkeit der Flüssigkeit durch die jeweilige Flussmesszelle anzeigt. Das Messwertpaar wird bevorzugt ein oder mehrmals pro Sekunde ermittelt und über Messleitungen 202 und 203 zu der Steuer- und Regeleinheit 101 übertragen. Die Steuer- und Regeleinheit 101 ordnet jedem Messwertpaar ein Volumenstrompaar zu, wobei eine Abbildung von einem Messwert auf einen Volumenstrom verwendet werden kann, die auf einer zuvor durchgeführten Kalibrierung beruht. Alternativ könnte auch eine Abbildung auf einen Massenstrom erfolgen. Die Steuer- und Regeleinheit 101 leitet aus dem ermittelten Volumenstrompaar ein Steuersignal für die Pumpe 211 ab, etwa so, dass die Pumpe 211 derart betrieben wird, dass der Volumenstrom durch beide Flussmesszellen 205 und 206 des Differenzstromsensors zu jedem Zeitpunkt übereinstimmt. Beispielsweise bildet die Steuer- und Regeleinheit 101 ein Differenzsignal aus beiden Volumenströmen des Volumenstrompaares und verändert die Flussrate der Ultrafiltrationspumpe 211 durch Erhöhung oder Verringerung je nach Vorzeichen des Differenzsignal in geeigneter Weise so, dass das Differenzsignal zu null verschwindet. Ist der Fluss durch die Flussmesszelle 205 kleiner als der Fluss durch die Flussmesszelle 206, so ergibt sich für die Differenz der Messwerte von Flussmesszelle 206 und Flussmesszelle 205 ein positiver Wert. Die Steuer- und Regeleinheit 101 kann nun das Steuersignal für Ultrafiltrationspumpe 211 so verändern, dass sich die Flussrate durch Ultrafiltrationspumpe 211 erhöht und der Fluss durch Flussmesszelle 206 bei unverändertem Fluss im Abfluss des Dialysators soweit verringert bis sich der gleiche Fluss wie durch Flussmesszelle 205 einstellt. Die Flussrate durch die Ultrafiltrationspumpe 211 zeigt dann den Differenzfluss zwischen dem aus der Dialysierflüssigkeitskammer austretenden Dialysierflüssigkeitsfluss und dem in die Dialysierflüssigkeitskammer eintretenden Dialysierflüssigkeitsfluss an. Die Flussrate durch die Ultrafiltrationspumpe 211 ist dann ein Maß für die Menge des im Dialysator 113 entzogenen Ultrafiltrats.

In einer Ausführungsform wird die Flussrate durch die Ultrafiltrationspumpe 211 auf einen vorbestimmten Wert eingestellt, und die Steuerung der Blutpumpe 115, der Dialysierflüssigkeitspumpe 107, sowie der variabel einstellbaren Drossel 117 erfolgt wie oben beschrieben so, dass der im Differenzstromsensor 201 gemessene Differenzstrom eine vorbestimmte Bedingung erfüllt, etwa: zu null verschwindet.

Hierbei ist die Flussrate durch die Ultrafiltrationspumpe 211 ein Maß für die Flüssigkeitsbilanz zwischen dem Zufluss zu dem Dialysator 113 und dem Abfluss aus dem Dialysator 113, d.h. für die Menge des im Dialysator 113 entzogenen Ultrafiltrats.

Das Verschwinden des Differenzsignals kann sich auf einen Differenzfluss zu einem bestimmten Zeitpunkt beziehen oder auf das Verschwinden eines Integrals eines Differenzflusses.

In einer weiteren Ausgestaltung kann auf die Zuordnung des Messwertpaars zu einem Volumen- oder Massenstrom verzichtet werden, wenn die Differenz der Messwerte bei gleichem Volumenstrom durch beide Kanäle bekannt ist. In diesem Fall bildet die Steuer- und Regeleinheit 101 die Differenz aus beiden Messwerten und verändert etwa die Durchgängigkeit der variabel einstellbaren Drossel 117 in geeigneter Weise solange bis die Differenz der vorbekannten Differenz bei gleichem Volumenstrom entspricht.

Der Differenzstromsensor 201 kann vorteilhaft nach dem magnetisch-induktiven Prinzip funktionieren. Dabei weisen die beiden Flussmesszellen 205, 206, die im Gegenstrom durchströmt werden, einen rechteckigen Querschnitt auf und werden rechtwinkelig zu einem Magnetfeld angeordnet. Das Magnetfeld wird dabei von der Steuerung des Differenzstromsensors 201 eingestellt und ist so beschaffen, dass durch beide Flussmesszellen 205, 206 ein homogenes und gleich großes Feld vorherrscht. Dies wird beispielsweise dadurch erreicht, dass die Kanäle der Flussmesszellen 205, 206 übereinander zum Magnetfeld angeordnet sind. In jedem Kanal ist gegenüberliegend und im rechten Winkel zum Magnetfeld und zur Flussrichtung im jeweiligen Kanal eine Elektrode an derjenigen inneren Kanalwand angebracht, die sich entlang des Magnetfeldes erstreckt. Strömt Flüssigkeit durch den Kanal, so wird durch das Magnetfeld eine Ladungstrennung der in der Flüssigkeit vorliegenden Ionen bewirkt, sodass an den Elektroden eine elektrische Spannung vorliegt. Diese Spannung ist proportional zu der Strömungsgeschwindigkeit und abhängig von der Magnetfeldstärke. Ist das Magnetfeld in den beiden Flussmesszellen 205 und 206 gleich groß, so fällt bei der Bildung eines Differenzsignals aus beiden Kanälen die Magnetfeldstärkeabhängigkeit für das relative Differenzflusssignal vorteilhaft weg.

Mit anderen Worten: ein Verschwinden des Differenzsignals zeigt unabhängig von der absoluten Größe des Magnetfelds in den Flussmesszellen 205 und 206 an, dass der Fluss durch die Flussmesszelle 205 und der Fluss durch die Flussmesszelle 206 gleich groß sind.

In der Ausführungsform, bei der für die Ultrafiltrationsrate oder für das Ultrafiltrationsvolumen ein Profil vorgegeben werden, bei dem sich Intervalle mit einer positiven Ultrafiltrationsrate und Intervalle mit einer negativen Ultrafiltrationsrate abwechseln, ist die vorbestimmte Bedingung vorteilhaft dann erfüllt, wenn das Integral der Ultrafiltrationsrate und/ oder des Differenzsignals zu Null verschwindet. Die Ultrafiltrationspumpe 211 ist bevorzugt aus der Gruppe der Verdrängerpumpen gewählt, bevorzugter eine Membran-, Schlauchrollen-, Kolben- oder Zahnradpumpe oder jede andere Pumpe die es erlaubt, die geförderte Flüssigkeitsmenge zu ermitteln. Beispielsweise kann mit der Schlauchrollenpumpe das geförderte Volumen durch das Pumpschlauchvolumen und dem Drehwinkel des Rotors der Schlauchrollenpumpe mit guter Genauigkeit mit bekannten Verfahren bestimmt werden. Auch für andere Pumpen aus der Gruppe der Verdrängerpumpen sind entsprechende Verfahren zur Bestimmung der geförderten Flüssigkeitsmenge aus dem Stand der Technik bekannt.

Vorteilhaft ist hierbei, dass die zu messende Flüssigkeitsmenge der Ultrafiltratmenge entspricht. Diese Menge liegt typischerweise bei 3-5l je Dialysebehandlung oder Tag, wohingegen die Menge an Dialysat, die durch den Flusssensor strömt, ein Vielfaches, typischerweise 60-240l davon beträgt. Im Einklang mit der vorliegenden Offenbarung ist es daher vorteilhaft möglich, Messgeräte oder Messverfahren für den Differenzfluss einzusetzen, die eine wesentlich geringere Toleranz aufweisen müssen, als dies Messverfahren müssten, die die abfließende und die zufließende Menge an Dialysat getrennt erfassen, und erst anschließend eine Differenzbildung vornehmen.

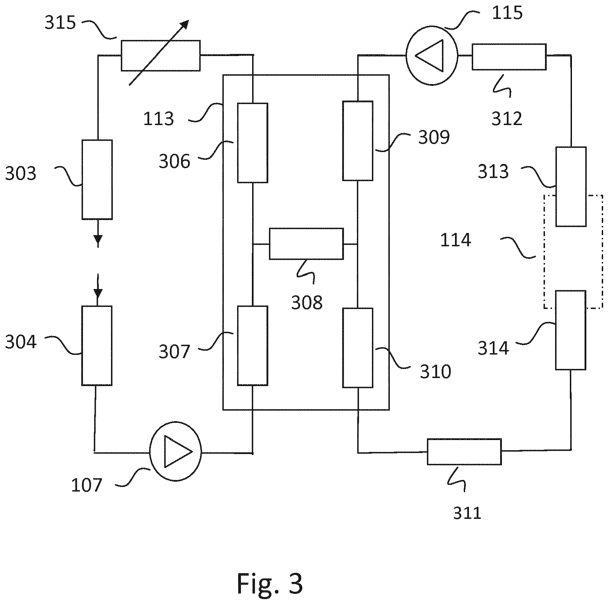

Figur 3 zeigt ein Ersatzschaltbild der in Figur 1 dargestellten Dialysevorrichtung mit der Dialysierflüssigkeitspumpe 107, der Blutpumpe 115, sowie dem Dialysator 113, wobei die Flusswiderstände im Dialysierflüssigkeitskreislauf, im Blutkreislauf sowie im Dialysator als Widerstände eines elektrischen Ersatzschaltbildes dargestellt sind. Im Einzelnen sind im extrakorporalen Blutkreislauf ein arterieller Nadelwiderstand 313, ein arterieller Leitungswiderstand 312, ein venöser Nadelwiderstand 314, ein venöser Leitungswiderstand 311, sowie im Dialysator 113 ein arterieller Filter-Längswiderstand 309, und ein venöser Filter-Längswiderstand 310 dargestellt. Im Dialysierflüssigkeitskreislauf werden die Flusswiderstände durch einen eingangsseitigen Flusswiderstand des Dialysators 307, einen ausgangsseitigen Flusswiderstand des Dialysators 306, einen Flusswiderstand 304 auf der Dialysateingangsseite des Dialysierflüssigkeitskreislaufs, einen fest vorgegebenen Flusswiderstand 303 auf der Dialysatausgangsseite des Dialysierflüssigkeitskreislaufs, sowie durch einen variablen Drosselwiderstand 315, d.h. den variablen Widerstand der einstellbaren Drossel 117 modelliert. Die Membran im Dialysator wird durch einen Transmembranwiderstand 308 modelliert. Für die unten angegebene Ableitung für eine Dimensionierung der Widerstände im Dialysierflüssigkeitskreislauf, im extrakorporalen Blutkreislauf sowie des Transmembranwiderstandes sind die Bezeichnungen der einzelnen Widerstände, ihre Bezugszeichen, sowie die in der Ableitung verwendeten Formelzeichen in der Tabelle 1 wiedergegeben.

Tabelle 1

| Bezeichnung des Widerstandes | Bezugszeich en | Formelzeichen |
|---|---|---|
| arterieller Nadelwiderstand im extrakorporalen Blutkreislauf | 313 | $R_{aN}$ |
| arterieller Leitungswiderstand im extrakorporalen Blutkreislauf | 312 | $R_{aL}$ |
| venöser Nadelwiderstand im extrakorporalen Blutkreislauf | 314 | $R_{vN}$ |
| venöser Leitungswiderstand im extrakorporalen Blutkreislauf | 311 | $R_{aN}$ |
| arterieller Filter-Längswiderstand | 309 | $R_{aF}$ |
| venöser Filter-Längswiderstand | 310 | $R_{vF}$ |
| eingangsseitiger Flusswiderstand im Dialysierflüssigkeitskreislauf | 304 | $R_{Din}$ |
| ausgangsseitiger Flusswiderstand im Dialysierflüssigkeitskreislauf | 303 | $R_{Dout}$ |
| eingangsseitiger Flusswiderstand des Dialysators | 307 | $R_{DFin}$ |
| ausgangsseitiger Flusswiderstand des Dialysators | 306 | $R_{DFout}$ |
| Transmembranwiderstand | 308 | $R_{TM}$ |
| Drosselwiderstand | 315 | $R_{Dr}$ |

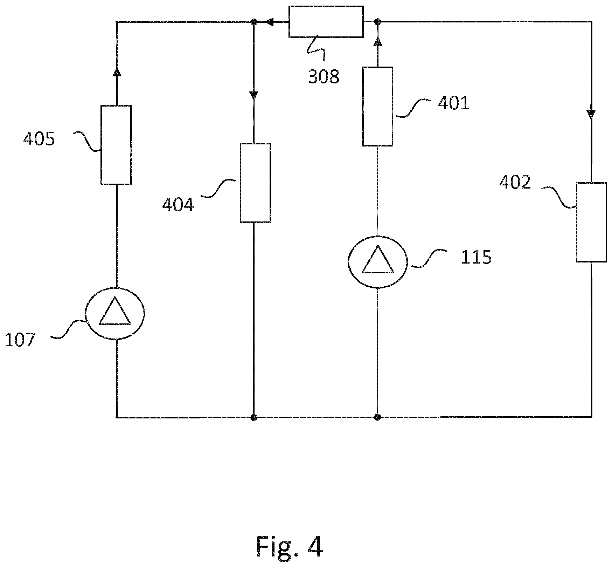

Ein vereinfachtes Ersatzschaltbild des in Figur 3 dargestellten elektrischen Ersatzschaltbildes zeigt Figur 4. In dem in Figur 4 dargestellten vereinfachten Ersatzschaltbild sind der arterieller Nadelwiderstand im extrakorporalen Blutkreislauf (Formelzeichen: $R_{aN}$), der arterielle Leitungswiderstand im extrakorporalen Blutkreislauf (Formelzeichen: $R_{aL}$) sowie der arterielle Filter-Längswiderstand (Formelzeichen: $R_{aF}$) zu einem arteriellen Gesamtwiderstand 401 (Formelzeichen: $R_a$) zusammengefasst:

$$R_a = R_{aN} + R_{aL} + R_{aF} \qquad \text{(Gleichung 1)}$$

Ebenso können der venöser Nadelwiderstand im extrakorporalen Blutkreislauf (Formelzeichen: $R_{vN}$) der venöse Leitungswiderstand (Formelzeichen: $R_{vL}$) im extrakorporalen Blutkreislauf, sowie der venöse Filter-Längswiderstand (Formelzeichen: $R_{vF}$) zu einem venösen Gesamtwiderstand 402 (Formelzeichen: $R_v$) zusammengefasst werden:

$$R_v = R_{vN} + R_{vL} + R_{vF} \qquad \text{(Gleichung 2)}$$

Eine entsprechende Zusammenfassung der Widerstände im Dialysierflüssigkeitskreislauf ergibt folgendes. Der eingangsseitige Flusswiderstand im Dialysierflüssigkeitskreislauf 304 (Formelzeichen: $R_{Din}$) und der eingangsseitige Flusswiderstand 307 des Dialysators (Formelzeichen: $R_{DFin}$) können zu einem Eingangswiderstand 405 (Formelzeichen: $R_{in}$) zusammengefasst werden:

$$R_{in} = R_{Din} + R_{DFin} \qquad \text{(Gleichung 3)}$$

Der ausgangsseitige Flusswiderstand im Dialysierflüssigkeitskreislauf 303 (Formelzeichen: $R_{Dout}$) und der ausgangsseitige Flusswiderstand des Dialysators 306 (Formelzeichen: $R_{DFout}$) können zu einem Ausgangswiderstand 404 (Formelzeichen: $R_{out}$) zusammengefasst werden:

$$R_{out} = R_{Dout} + R_{DFout} + R_{Dr} \qquad \text{(Gleichung 4).}$$

Die Bezeichnungen der in Figur 4 dargestellten Widerstände, ihrer Bezugszeichen, sowie die in der Ableitung verwendeten Formelzeichen sind in der folgenden Tabelle 2 widergegeben.

Tabelle 2

| Bezeichnung des Widerstandes | Bezugzeichen | Formelzeichen |
|---|---|---|
| arterieller Gesamtwiderstand | 401 | $R_a$ |
| venöser Gesamtwiderstand | 402 | $R_v$ |
| Eingangswiderstand (im Dialysierflüssigkeitskreislauf) | 405 | $R_{in}$ |
| Ausgangswiderstand (im Dialysierflüssigkeitskreislauf) | 404 | $R_{out}$ |
| Transmembranwiderstand | 308 | $R_{TM}$ |

Die Blutpumpe und die Dialysierflüssigkeitspumpe können als Stromquelle oder als Spannungsquelle modelliert werden, wobei die geeignete Modellierung von der Bauart der Pumpe beeinflusst wird. So ist bei der Verwendung einer Verdrängerpumpen, etwa einer Membran-, Schlauchrollen-, Kolben- oder Zahnradpumpe als Dialysierflüssigkeitspumpe die Modellierung der Dialysierflüssigkeitspumpe als Stromquelle vorteilhaft. Entsprechendes gilt, wenn die Blutpumpe als Verdrängerpumpe, etwa als Schlauchrollenpumpe ausgeführt ist. Eine druckkonstante Pumpe wie etwa eine Impellerpumpe wird vorzugsweise als Spannungquelle modelliert. Werden die Blutpumpe oder die Dialysierflüssigkeitspumpe als nicht-ideale Spannungs- oder Stromquelle mit entsprechenden Innenwiderständen modeliert, so müssen die jeweiligen Innenwiderstände den Widerständen im Dialysierflüssigkeitskreislauf beziehungsweise im extrakorporalen Blutkreislauf noch zugeschlagen werden. So muss etwa bei der Modellierung der Dialysierflüssigkeitspumpe als nicht-ideale Spannungsquelle der Strömungwiderstand der Dialysierflüssigkeitspumpe in den Eingangswiderstand 405 aufgenommen werden. Entsprechendes gilt für den extrakorporalen Blutkreislauf. Dem Fachmann sind die entsprechenden hierzu erforderlichen Überlegungen bekannt. Dem Fachmann ist ebenfalls bekannt wie Ersatzschaltbilder, bei denen nicht-ideale Spannungsquellen modelliert werden in entsprechende Ersatzschaltbilder mit nicht-idealen Stromquellen umzuwandeln sind.

Bei der Dimensionierung der Widerstände im extrakorporalen Blutkreislauf und im Dialysierflüssigkeitskreislauf sowie bei der Dimensionierung der Innenwiderstände der beteiligten Pumpen, und beim Ansteuern der beteiligten Pumpen zum Erzielen einer gewünschten Ultrafiltrationsrate können folgende Überlegungen hilfreich sein. Wenn für die Ultrafiltrationsrate ein entsprechender Strom $I_{UF}$ im elektrischen Ersatzschaltbild angesetzt wird, so kann im Fall der Modellierung der Pumpen als Spannungsquellen, wenn die Dialysierflüssigkeitspumpe mit einer Spannungsquelle der Spannung $U_D$ und die Blutpumpe mit einer Spannungsquelle der Spannung $U_B$ modelliert wird, folgende Formel für den die Ultrafiltrationsrate angegeben werden:

$$I_{UF} = \frac{\dfrac{U_B \cdot R_V}{R_a + R_V} - \dfrac{U_D \cdot R_{out}}{R_{in} + R_{in}}}{\dfrac{R_a \cdot R_v}{R_a + R_v} + \dfrac{R_{in} \cdot R_{out}}{R_{in} + R_{out}} + R_{TM}} \qquad \text{(Gleichung 5)}$$

Im Fall der Modellierung der Pumpen als Stromquellen kann, wenn die Dialysierflüssigkeitspumpe mit einer Stromquelle des Stroms $I_D$ und die Blutpumpe mit einer Stromquelle des Stroms $I_B$ modelliert wird, folgende Formel für die Ultrafiltrationsrate angegeben werden:

$$I_{UF} = \frac{I_B \cdot R_V - I_D R_D}{R_v + R_{out} + R_{TM}} \qquad \text{(Gleichung 6)}$$

Für die Dimensionierung des Ausgangswiderstandes 404 (Formelzeichen $R_{out}$) ist folgende Überlegung hilfreich. Die Umstellung der Formel 6 für den zum Erzielen einer bestimmten Ultrafiltrationsrate erforderlichen Ausgangswiderstand 404 (Formelzeichen $R_{out}$) ergibt folgende Beziehung:

$$R_{out} = \frac{R_v \cdot (I_B - I_{UF}) - R_{TM} I_{UF}}{I_D - I_{UF}} \qquad \text{(Gleichung 7)}$$

Die Formel 7 zeigt, dass ein zu hoher Transmembranwiderstand $R_{TM}$ einen ungünstigen Einfluss auf die Di-

mensionierung des Ausgangswiderstands $R_{out}$ im Dialysierflüssigkeitskreislauf hat. Der Transmembranwiderstand $R_{TM}$ sollte daher möglichst klein gewählt werden, etwa als Filter mit einem hohen spezifischen Durchgangskoeffizienten ("high Cut-off Filter") oder als Filter mit einer ausreichend großen effektiven Filterfläche. Dabei ist zu berücksichtigen, dass der Transmembranwiderstand $R_{TM}$ sich ebenso wie der venöse Filter-Längswiderstand $R_{vF}$ im Verlauf der extrakorporalen Blutbehandlung zunehmen werden. Die Zunahme des venösen Filter-Längswiderstandes $R_{vF}$ im extrakorporalen Blutkreislauf beruht typischerweise auf der Zunahme des Hämatokrits im Laufe der Blutbehandlung, der sogenannten Hämokonzentration, sowie auf einem möglichen Entstehen von Engstellen im extrakorporalen Blutkreislauf. Eine Zunahme des Transmembranwiderstandes $R_{TM}$ im Laufe einer Blutbehandlung entsteht häufig durch Ablagerungen an der Dialysatormembran. Diese Effekte, die sich regelmäßig im Laufe einer Blutbehandlung einstellen, müssen bei der Dimensionierung der Widerstände im extrakorporalen Blutkreislauf und im Dialysierflüssigkeitskreislauf sowie bei der Ansteuerung der Blutpumpe, der Dialysierflüssigkeitspumpe sowie der variabel einstellbaren Drossel berücksichtigt werden.

Folgendes Zahlenbeispiel kann einen Anhaltspunkt für eine mögliche Dimensionierung der beteiligten Flüsse geben, d.h. für den Blutflusse $I_B$, wird von einem Minimalwert $I_{Bmin}$ = 60 ml/min und einem Maximalwert $I_{\beta max}$ = 300 ml/min ausgegangen wird, für die Ultrafiltrationsrate $I_{UF}$ von einem Maximalwert $I_{UFmax}$ = $I_B$/ 10, also etwa 20 ml/min und einem Minimalwert $I_{UFmin}$ = 0 ml/min sowie für die Dialysierflüssigkeitsrate $I_D$ von einem Minimalwert $I_{Dmin}$ = $I_B$/ 3 und einem Maximalwert $I_{Dmax}$ = 200 ml/min.

Der Blutfluss $I_B$ ist dabei als der Fluss durch die Blutpumpe 115 gesetzt, der Dialysierflüssigkeitsrate $I_D$ entspricht ist dem Fluss durch die Dialysierflüssigkeitspumpe 107 und die Ultrafiltrationsrate $I_{UF}$ entspricht dem Fluss durch den Transmembranwiderstand 308.

Für die Dialysierflüssigkeitsrate $I_D$ gilt allgemein folgende Beziehung:

$$I_D = \frac{I_B \cdot R_V - I_{UF} \cdot (R_V + R_{out} + R_{TM})}{R_{out}} \hspace{3cm} \text{(Gleichung 8)}$$

Grundsätzlich ergibt sich bei einem vorgegebenen Blutfluss $I_B$, dass eine maximale Ultrafiltration dann erzielt wird, wenn die Dialysierflüssigkeitsrate $I_D$ minimal ist.
Nach einer Umstellung ergibt sich so aus Gleichung 8 folgende Beziehung:

$$I_{D\min} \leq \frac{R_V}{R_{out}} \cdot (I_B - I_{UF}) - \left(1 + \frac{R_{TM}}{R_{out}}\right) I_{UF} \hspace{2cm} \text{(Gleichung 9)}$$

Setzt man als alternative Zahlenwerte für den minimalen Blutfluss $I_B$ = 50 ml/min ein und für die Ultrafiltrationsrate $I_{UF}$ = 5 ml/min, so ergibt sich die folgende Beziehung, die die Größen der Widerstände $R_V$, $R_{out}$ und $R_{TM}$ zu einander in eine Beziehung setzt:

$$20 \leq \frac{R_V}{R_{out}} \cdot (50 - 5) - \left(1 + \frac{R_{TM}}{R_{out}}\right) 5 \hspace{2cm} \text{(Gleichung 10)}$$

oder:

$$25 \leq 45 \cdot \frac{R_V}{R_{out}} - 5 \cdot \frac{R_{TM}}{R_{out}} \hspace{3cm} \text{(Gleichung 11)}$$

Ausgehend von einem bestimmten Verhältnis zwischen dem Transmembranwiderstand $R_{TM}$ und dem Ausgangswiderstand im Dialysierflüssigkeitskreislauf, ergeben sich die folgenden Beziehungen für die Dimensionierung des Ausgangswiderstands $R_{out}$ im Dialysierflüssigkeitskreislauf in Bezug zu dem venösen Gesamtwiderstand $R_V$, wobei aufsteigende Werte für den Transmembranwiderstand $R_{TM}$ angegeben sind, die die oben angesprochenen Effekte eines Anstiegs über den Behandlungsverlauf wiederspiegeln.

$$R_{TM} = R_{out} / 2 \qquad \rightarrow \qquad R_{out} \leq \frac{90}{55} R_V$$

$$R_{TM} = R_{out} \qquad \rightarrow \qquad R_{out} \leq \frac{45}{30} R_V$$

$$R_{TM} = 2 \cdot R_{out} \qquad \rightarrow \qquad R_{out} \leq \frac{45}{35} R_V$$

$$R_{TM} = 4 \cdot R_{out} \qquad \rightarrow \qquad R_{out} \leq \frac{45}{45} R_V$$

Diese Beispielrechnung zeigt, dass der Ausgangswiderstand $R_{out}$ umso kleiner gewählt werden muss, wenn der Transmembranwiderstand $R_{TM}$ größer ist. Wie bereits oben angesprochen ist für die Auslegung der Flusswiderstände im Dialysierflüssigkeitskreislauf und im extrakorporalen Blutkreislauf ein zu hoher Transmembranwiderstand $R_{TM}$ nachteilig. Nimmt man beispielsweise an, das der Transmembranwiderstand $R_{TM}$ als Maximalwert das Vierfache des Ausgangswiderstands $R_{out}$ im Dialysierflüssigkeitskreislauf annehmen kann, so ergibt das oben angeführte Zahlenbeispiel die einfache Forderung, dass $R_{out}$ kleiner als $R_V$ sein muss. Für die Auslegung des Ausgangswiderstands $R_{out}$ im Dialysierflüssigkeitskreislauf und des venösen Gesamtwiderstandes $R_V$ ist es ausreichend, den venösen Gesamtwiderstand $R_V$ zu Beginn der Blutbehandlung anzusetzen, da mit einem während der Blutbehandlung ansteigenden venösen Gesamtwiderstand die Gleichung 9 umso eher erfüllt sein wird.

Die Steuerung des Ausgangswiderstands $R_{out}$ erfolgt in dem Ausführungsbeispielen der Figuren 1 und 2 durch eine entsprechende Einstellung der Drossel 117.

Für die nachfolgende beispielhafte Angabe von Werten für die Rate des Dialysierflüssigkeitsflusses $I_D$, des Blutflusses $I_B$, sowie der Ultrafiltrationsrate $I_{UF}$ wird davon ausgegangen, dass zu Beginn der Behandlung der Wert des Ausgangswiderstandes $R_{out}$ dem venösen Gesamtwiderstand $R_V$ entspricht und dass der Transmembranwiderstand $R_{TM}$ zu Beginn der Behandlung dem Ausgangswiderstandes $R_{out}$ entspricht.

Bei einem Blutfluss $I_B$ = 200 ml/min, wird ausgehend von Gleichung 9 die maximale Ultrafiltrationsrate $I_{UF}$ = 20 ml/min bei einer Dialysierflüssigkeitsrate

$$I_D = (200 - 20)ml/\min - 2 \cdot 20 ml/\min = 140 ml/\min$$

erzielt.

Die minimale Ultrafiltrationsrate $I_{UF}$ = 0 ml entspricht einer Dialysierflüssigkeitsrate

$$I_D = (200 - 0)ml/\min - 2 \cdot 0 ml/\min = 200 ml/\min$$

Beide Werte liegen in einem zulässigen, akzeptablen oder bevorzugten Bereich der Dialysierflüssigkeitsrate $I_D$ < 200 ml/min.

Für das folgende Zahlenbeispiel wird davon ausgegangen, dass im weiteren Verlauf der Blutbehandlung der Transmembranwiederstand $T_M$ in Folge der oben angesprochenen Effekte auf den vierfachen Wert ansteigt. Um eine maximale Ultrafiltrationsrate $I_{UF}$ = 20 ml/min zu erzielen, wäre eine Drosselung der Dialysierflüssigkeitsrate auf

$$I_D = (200 - 20)ml/\min - 5 \cdot 20 ml/\min = 80 ml/\min$$

erforderlich.

Für die minimale Ultrafiltrationsrate $I_{UF}$ = 0 ml wäre nach wie vor eine Dialysierflüssigkeitsrate

$$I_D = (200 - 0)ml/\min - 5 \cdot 0 ml/\min = 200 ml/\min \quad \text{einzustellen.}$$

Nimmt man an, dass sich in Folge der oben angesprochenen Effekte der venöse Gesamtwiderstand $R_V$ im Laufe der Blutbehandlung verdoppelt, so ergibt sich für eine maximale zu erzielende Ultrafiltrationsrate von 20 ml/min ein erforderlicher Dialysierflüssigkeitsfluss von

$$I_D = 2 \cdot (200 - 20)ml \, / \min - 5 \cdot 20 ml \, / \min = 260 ml \, / \min \, .$$

Für eine minimale Ultrafiltrationsrate $I_{UF}$ = 0 ml ergibt sich ein Dialysierflüssigkeitsfluss von

$$I_D = 2 \cdot (200 - 0)ml \, / \min - 5 \cdot 0 ml \, / \min = 400 ml \, / \min \, .$$

Um in diesem Fall die Ultrafiltrationsrate über den gesamten Bereich von 0 bis 20 ml/min regeln zu können, besteht die Möglichkeit, den als zulässig, akzeptabel oder bevorzugt angesehenen Bereich des Dialysierflüssigkeitsflusses zu erweitern oder den Blutfluss $I_B$ zu verringern. So würde etwa bei einem maximal zulässigen Dialysierflüssigkeitsfluss $I_D$ = 200 ml/min, eine maximale Ultrafiltrationsrate $I_{UF}$ = 20 ml/min bei einem Blutfluss $I_B$ = 170 ml/min erzielt, und eine minimale Ultrafiltrationsrate $I_{UF}$ = 0 ml/min bei einem Blutfluss $I_B$ = 100 ml/min.

Das folgende Rechenbeispiel soll verdeutlichen, wie vorteilhaft es ist, den Transmembranwiderstand $R_{TM}$ gering zu halten. Wenn man etwa durch die Dimensionierung des Dialystors und des Dialysierflüssigkeitskreislaufs sicherstellt, dass der Transmembranwiderstand $R_{TM}$ maximal dem Ausgangswiderstand $R_{out}$ im Dialysierflüssigkeits-kreislauf entspricht, und legt man den Dialysierflüssigkeitskreislauf und den extrakorporalen Blutkreislauf so aus, dass der Ausgangswiderstand $R_{out}$ im Dialysierflüssigkeitskreislauf zu dem venösen Gesamtwiderstand in einem Verhältnis $R_{out}$ = 3/2 $R_V$ ist, so ergibt sich bei einem Blutfluss $I_B$ = 200 ml/min eine maximale Ultrafiltrationsrate von 20 ml/min bei einer Dialysierflüssigkeitsrate

$$I_D = 4 \, / \, 3 \cdot (200 - 20)ml \, / \min - 2 \cdot 20 ml \, / \min = 200 ml \, / \min$$

und bei demselben Blutfluss $I_B$ eine minimale Ultrafiltrationsrate von 0 ml/min bei einer Dialysierflüssigkeitsrate

$$I_D = 4 \, / \, 3 \cdot (200 - 0)ml \, / \min - 2 \cdot 0 ml \, / \min = 267 ml \, / \min \, .$$

Der Bereich einer Ultrafiltrationsrate $I_{UF}$ von 0 ml/ min bis 20 ml/min kann somit mit einer geringeren Variation der Dialysierflüssigkeitsrate $I_D$ angesteuert werden.

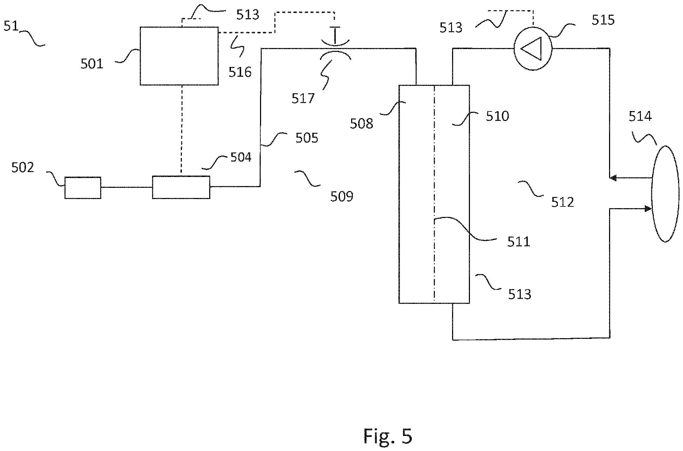

In Figur 5 ist schematisch in einem nicht zur Erfindung gehörenden Beispiel ein Hämofiltrationsgerät 51 mit einer Vorrichtung zur Regelung der Hämofiltration dargestellt. Das zu behandelnde Blut wird über einen Zugang 514 dem Patienten entnommen und mit einer Blutpumpe 515 im extrakorporalen Blutkreislauf 512 durch eine Blutkammer des Dialysators oder Hämofilters 513 und über den Zugang 514 an den Patienten zurückgegeben. Der Zugang 514 verbindet den Blutkreislauf 512 mit einem Blutgefäß des Patienten, das zur Blutentnahme und Rückgabe geeignet ist. Der Zugang 514 kann zur Blutentnahme und zur Rückgabe des Blutes einen getrennten Ab- und Zufluss enthalten (‚Double Needle'- Verfahren), oder Zu- und Abfluss können als ein Element ausgeführt sein (‚Single Needle'- Verfahren). Stromauf oder stromab des Dialysators oder Hämofilters 513 erfolgt im Blutkreislauf 512 eine Zugabe von Substitutionsflüssigkeit durch eine Substituatleitung (nicht gezeigt).

Im Dialysator oder Hämofilter 513 trennt eine semipermeable Membran 511 eine Filtratkammer 508 von einer Blutkammer 510. Über die semipermeable Membran 511 findet ein Flüssigkeits- und Stoffaustausch von der Blutkammer 510 in die Filtratkammer 108 statt. Der Filtratfluss in der Ableitung 505 aus der Filtratkammer 508 wird mittels der variabel einstellbaren Drossel 517 kontrolliert. Aus dem Dialysator oder Hämofilter 513 abfließendes Filtrat wird wird im Hinblick auf den Filtratfluss von einem Flussmesser 504 erfasst und im Allgemeinen in einen Filtratabfluss 502 verworfen.

Durch die Steuerung der Blutpumpe 515, und/oder die Steuerung der variabel einstellbaren Drossel 517 werden die Druckverhältnisse an der Membran 511 im Hämofilter 513 so beeinflusst, dass in der Blutkammer 510 gegenüber der Filtratkammer 508 ein Überdruck herrscht. Dadurch erfolgt ein Transport von Flüssigkeit durch die Membran von der Blutkammer 510 in die Filtratkammer 508.

Die Blutpumpe kann zum Erzielen einer bestimmten Pumpendrehzahl oder einer bestimmten Blutflussrate als Betriebsparameter ansteuerbar sein, etwa in einer Ausführung als peristaltische Pumpe. Alternativ kann die Blutpumpe zum Erzielen eines bestimmten Förderdrucks als Betriebsparameter ansteuerbar sein, etwa als Impellerpumpe.

Die variable einstellbare Drossel 517 kann als Drossel mit einem variabel einstellbaren Querschnitt als Durchfluss, als getaktetes Ventil oder als anderes Bauelement ausgeführt sein, dessen Fluidwiderstand variabel einstellbar ist, einschließlich eines variabel einstellbaren Fluidwiderstandes, der sich erst durch eine zeitliche Mittelung ergibt.

Eine mit dem Flussmesser 504 über eine Messleitung verbundene Steuer- und Regeleinheit 501 ist über die Steuerleitung 513 mit der Blutpumpe 515 über die Steuerleitung 516 mit der variabel einstellbaren Drossel 517 verbunden. Während der Blutbehandlung werden kontinuierlich oder periodisch aktuelle Messparameter der Hämofiltration, etwa der Filtratmenge oder Filtrationsrate von dem Flussmesser 504 an die Steuer- und Regeleinheit 501 übermittelt. Die Steuer- und Regeleinheit 501 verwendet die aktuellen Messparameter um Steuersignale für die die Blutpumpe 515, sowie für die variabel einstellbare Drossel 517 abzuleiten. Die Ansteuerung der Blutpumpe 515 und der variabel einstellbaren Drossel 517 erfolgt dabei im Hinblick auf eine zu erzielende Ultrafiltration, etwa eine bestimmte Filtrationsrate oder ein bestimmtes, über den Behandlungsverlauf zu erzielendes Filtratvolumen.

Die Regelung kann etwa so erfolgen, dass die Blutpumpe 515 mit einer konstanten Drehzahl oder mit einem konstanten Förderdruck betrieben wird, und die variabel einstellbare Drossel 517 so gesteuert wird, dass der von dem Flussmesser 504 übermittelte Messwert als Regelgröße dient. Liegt etwa der übermittelte gemessene Wert der Filtrationsrate über einem entsprechenden Sollwert, so ist die variabel einstellbare Drossel 117 zu verengen, oder mit anderen Worten: ihr Flusswiderstand ist zu erhöhen, liegt die Filtrationsrate unter ihrem Sollwert, wird die variabel einstellbare Drossel 517 geweitet, oder mit anderen Worten: ihr Flusswiderstand wird vermindert.

Eine alternative Regelstrategie ist derart, dass die variabel einstellbare Drossel 117 auf einen bestimmten Flusswiderstand eingestellt wird und die Blutpumpe 515 so gesteuert wird, dass der von dem Flussmesser 504 übermittelte Filtrationswert als Regelgröße dient. Liegt etwa der übermittelte Wert der Filtrationsrate über einem entsprechenden Sollwert, so ist die Blutpumpe 507 zu drosseln, liegt die Filtrationsrate unter ihrem Sollwert, wird die Blutpumpe 507 beschleunigt.

Die Regelung der Filtration kann so erfolgen, dass für die Filtrationsrate ein bestimmter Wert vorgegeben wird. Alternativ kann für das während der Blutbehandlung zu entziehende Filtrationsvolumen ein bestimmtes Filtrationsprofil vorgegeben werden.

Anstelle der Filtratmenge kann die während der Behandlung über die Substituatleitung zugefügte Substituatmenge zusammen mit dem entzogenen Filtrat in eine Bilanz eingestellt werden und die resultierende Ultrafiltrationsrate, d.h. die Differenz der Filtrationsrate und der Substituatrate als Regelgröße herangezogen werden.

Der vorgegebene Wert der Ultrafiltrationsrate kann ein konstanter oder sich kontinuierlich ändernder Wert für die Ultrafiltrationsrate sein.

Die Regelung der Ultrafiltration kann in diesem Fall analog zu den oben für die Ultrafiltrationsrate beschriebenen Regelstrategien erfolgen, wobei an die Stelle eines Abgleichs mit dem Sollwert der Ultrafiltrationsrate ein entsprechender Abgleich mit einem Ultrafiltrationsprofil erfolgt.

## Patentansprüche

1. Vorrichtung (1, 2) zur Regelung einer Ultrafiltration bei einer Dialysebehandlung bei der zu ultrafiltrierendes Blut in einem extrakorporalen Blutkreislauf (112) eine Blutkammer (110) eines durch eine semipermeable Membran (111) in die Blutkammer (110) und eine Dialysierflüssigkeitskammer (108) unterteilten Dialysators (113) durchströmt und Dialysierflüssigkeit in einem Dialysierflüssigkeitskreislauf (109) die Dialysierflüssigkeitskammer (108) des Dialysators (113) durchströmt,
   mit einer Blutpumpe (115) zur Kontrolle eines Blutflusses in dem extrakorporalen Blutkreislauf (112), mit einer in dem Dialysierflüssigkeitskreislauf (109) stromauf des Dialysators (113) angeordneten Dialysierflüssigkeitstpumpe (107) zur Kontrolle eines Dialysierflüssigkeitsflusses in einem Zustrom zur Dialysierflüssigkeitskammer, mit einer in dem Dialysierflüssigkeitskreislauf stromab des Dialysators angeordneten Drossel (117) zur Kontrolle des Dialysierflüssigkeitsflusses in einem Abfluss aus der Dialysierflüssigkeitskammer (108),
   mit einer Bilanziervorrichtung (104) zum Aufstellen einer Flüssigkeitsbilanz im Dialysierflüssigkeitskreislauf zwischen dem Zustrom (106) und dem Abfluss (105) der Dialysierflüssigkeitskammer (108) als Maß für die Ultrafiltration, und mit einer Regeleinheit (101), **dadurch gekennzeichnet dass** die Regeleinheit konfiguriert ist, die Drossel (117) so zu regeln, dass eine vorbestimmte Ultrafiltration erzielt wird.

2. Vorrichtung nach Anspruch 1, wobei die Regeleinheit (101) weiterhin zum Regeln der Blutpumpe (115) und der (107) Dialysierflüssigkeitspumpe angepasst ist, so dass eine vorbestimmte Ultrafiltration erzielt wird.

3. Vorrichtung (1, 2) zur Regelung einer Ultrafiltration bei einer Dialysebehandlung bei der zu ultrafiltrierendes Blut in einem extrakorporalen Blutkreislauf (112) eine Blutkammer (110) eines durch eine semipermeable Membran (111) in die Blutkammer (110) und eine Dialysierflüssigkeitskammer (108) unterteilten Dialysators (113) durchströmt und Dialysierflüssigkeit in einem Dialysierflüssigkeitskreislauf (109) die Dialysierflüssigkeitskammer (108) des Dialysators (113) durchströmt,

mit einer Blutpumpe (115) zur Kontrolle eines Blutflusses in dem extrakorporalen Blutkreislauf (112), mit einer in dem Dialysierflüssigkeitskreislauf (109) stromab des Dialysators (113) angeordneten Dialysierflüssigkeitspumpe zur Kontrolle eines Dialysierflüssigkeitsflusses in einem Abfluss aus der Dialysierflüssigkeitskammer,

mit einer in dem Dialysierflüssigkeitskreislauf stromauf des Dialysators angeordneten Drossel zur Kontrolle des Dialysierflüssigkeitsflusses in einem Zustrom zur Dialysierflüssigkeitskammer,

mit einer Bilanziervorrichtung (104) zum Aufstellen einer Flüssigkeitsbilanz im Dialysierflüssigkeitskreislauf zwischen dem Zustrom (106) und dem Abfluss (105) der Dialysierflüssigkeitskammer (113) als Maß für die Ultrafiltration, und mit einer Regeleinheit (101), **dadurch gekennzeichnet, dass** die Regeleinheit konfiguriert ist, die Drossel so zu regeln, dass eine vorbestimmte Ultrafiltration erzielt wird.

4. Vorrichtung nach Anspruch 3, wobei die Regeleinheit (101) weiterhin zum Regeln der Blutpumpe (115) und der Dialysierflüssigkeitspumpe angepasst ist. so dass eine vorbestimmte Ultrafiltration erzielt wird.

5. Vorrichtung (1, 2) nach einem der Ansprüche 2 oder 4, wobei für den Blutfluss ein vorbestimmter Wert einstellbar ist und die Regeleinheit angepasst ist zur Regelung des Dialysierflüssigkeitsflusses in dem Zustrom und/ oder in dem Abfluss der Dialysierflüssigkeitskammer.

6. Vorrichtung (1, 2) nach einem der Ansprüche 2 oder 4, wobei für den Dialysierflüssigkeitsfluss ein vorbestimmter Wert einstellbar ist und die Regeleinheit angepasst ist zur Regelung des Blutflusses.

7. Vorrichtung (2) nach einem der vorangehenden Ansprüche, wobei die Bilanziervorrichtung eine Differenzflussmesseinheit (104) zum Messen des Differenzflusses zwischen einem Fluss in dem Zustrom zur Dialysierflüssigkeitskammer (108) und dem Abfluss aus der Dialysierflüssigkeitskammer, eine Verzweigung von dem Zufluss oder dem Abfluss zur Abzweigung von Dialysierflüssigkeit von dem Zufluss oder dem Abfluss in einen weiteren Flussweg (212), sowie eine Einrichtung zur Einstellung der Flussmenge (211) in dem Zufluss, in dem Abfluss und/ oder in dem weiteren Flussweg, aufweist, die derart ansteuerbar ist, dass der gemessene Differenzfluss eine vorbestimmte Bedingung erfüllt, und mit einer Einrichtung (211) zur Ermittlung der Flussmenge in dem weiteren Flussweg als Maß der Flüssigkeitsbilanz.

8. Vorrichtung (1, 2) nach einem der Ansprüche 1 - 6, wobei durch die Regeleinheit (101) ein Profil für die Ultrafiltrationsrate vorgebbar ist, bei dem sich Intervalle mit einer positiven Ultrafiltrationsrate und Intervalle mit einer negativen Ultrafiltrationsrate abwechseln.

9. Vorrichtung (2) nach Anspruch 7 sowie Anspruch 8 wobei die vorbestimmte Bedingung des Differenzflusses auf einer Integration des Differenzflusses über ein vorbestimmtes Integrationsintervall beruht.

10. Vorrichtung (1, 2, 51) nach einem der vorangehenden Ansprüche, wobei die Regeleinheit (101) angepasst ist, eine Ultrafiltrationsrate und/oder ein während eines Behandlungsverlaufs zu entziehendes Ultrafiltrationsvolumen oder Hämofiltrationsvolumen vorzugegeben.

11. Vorrichtung (1, 2, 51) nach einem der vorangehenden Ansprüche, wobei die Blutpumpe in einer Zuleitung zu der Blutkammer angeordnet ist.

12. Vorrichtung (1, 2, 51) nach einem der vorangehenden Ansprüche, wobei die Drossel (117) als getaktetes Ventil ausgeführt ist.

**Claims**

1. A device (1, 2) for regulating ultrafiltration in a dialysis treatment, in which blood to be ultrafiltered flows through a blood chamber (110) of a dialyzer (113) that is subdivided by a semipermeable membrane (111) into the blood chamber (110) and a dialysis fluid chamber (108) in an extracorporeal blood circulation (112), and dialysis fluid flows through the dialysis fluid chamber (108) of the dialyzer (113) in a dialysis fluid circulation (109),

having a blood pump (115) for controlling the blood flow in the extracorporeal blood circulation (112), having a dialysis fluid pump (107) situated upstream from the dialyzer (113) in the dialysis fluid circulation (109) for controlling a dialysis fluid flow in an inflow to the dialysis fluid chamber, having a throttle (117) situated downstream from the dialyzer in the dialysis fluid circulation for controlling the dialysis fluid flow in an outflow out of the dialysis fluid chamber (108),
having a balancing device (104) for establishing a fluid balance in the dialysis fluid circulation between the inflow (106) and the outflow (105) of the dialysis fluid chamber (108) as a measure of ultrafiltration, and having a regulating unit (101), **characterized in that**
the regulating unit is configured to regulate the throttle (117), so that a predetermined ultrafiltration is achieved.

2. The device according to claim 1,
wherein the regulating unit (101) is also adapted for regulating the blood pump (115) and the dialysis fluid pump (107), so that a predetermined ultrafiltration is achieved.

3. The device (1, 2) for regulating ultrafiltration in a dialysis treatment, in which blood to be ultrafiltered flows through a blood chamber (110) of a dialyzer (113), which is subdivided by a semipermeable membrane (111) into a blood chamber (110) and a dialysis fluid chamber (108), in an extracorporeal blood circulation (112), and dialysis fluid flows through the dialysis fluid chamber (108) of the dialyzer (113) in a dialysis fluid circulation (109), comprising a blood pump (115) for control of the blood flow in the extracorporeal blood circulation (112), and comprising a dialysis fluid pump situated downstream from the dialyzer (113) in the dialysis fluid circulation (109) for control of the dialysis fluid flow in an outflow out of the dialysis fluid chamber,
having a throttle situated in the dialysis fluid circulation downstream from the dialyzer for control of the dialysis fluid flow in an inflow to the dialysis fluid chamber,
having a balancing device (104) for establishing a fluid balance in the dialysis fluid circulation between the inflow (106) and the outflow (105) of the dialysis fluid chamber (113) as a measure of the ultrafiltration, and having a regulating unit (101), **characterized in that**
the regulating unit is configured to regulate the throttle, so that a predetermined ultrafiltration is achieved.

4. The device according to claim 3,
wherein the regulating unit (101) is also adapted for regulating the blood pump (115) and the dialysis fluid pump, so that a predetermined ultrafiltration is achieved.

5. The device (1, 2) according to any one of claims 2 or 4,
wherein a predetermined value can be set for the blood flow, and the regulating unit is adapted for regulating the dialysis fluid flow in the inflow and/or in the outflow of the dialysis fluid chamber.

6. The device (1, 2) according to any one of claims 2 or 4,
wherein a predetermined value can be set for the dialysis fluid flow, and the regulating unit is adapted for regulating the blood flow.

7. The device (2) according to any one of the preceding claims,
wherein the balancing device has a differential flow measuring unit (104) for measuring the differential flow between the flow in the inflow to the dialysis fluid chamber (108) and the outflow out of the dialysis fluid chamber, a branch from the inflow or from the outflow for diverting dialysis fluid from the inflow or the outflow into another flow path (212) as well as a device (211) for adjusting the flow rate in the inflow, in the outflow and/or in the additional flow path, which can be controlled in such a way that the measured differential flow satisfies a predetermined condition, and having a device (211) for determining the flow rate in the additional flow path as a measure of the liquid balance.

8. The device (1, 2) according to any one of claims 1 through 6,
wherein a profile for the ultrafiltration rate can be preselected by the regulating unit (101), in which intervals with a positive ultrafiltration rate alternate with intervals with a negative ultrafiltration rate.

9. The device (2) according to claim 7 and claim 8,
wherein the predetermined condition of the differential flow is based on integration of the differential flow over a predetermined integration interval.

10. The device (1, 2, 51) according to any one of the preceding claims,
wherein the regulating unit (101) is adapted to predefine an ultrafiltration rate and/or an ultrafiltration volume or a hemofiltration volume to be withdrawn during the course of a treatment.

11. The device (1, 2, 51) according to any one of the preceding claims,
wherein the blood pump is situated in a feeder line to the blood chamber.

**12.** The device (1, 2, 51) according to any one of the preceding claims,
wherein the throttle (117) is embodied as a cycled valve.

**Revendications**

**1.** Dispositif (1, 2) de régulation d'une ultrafiltration dans un traitement par dialyse, dans lequel le sang à ultrafiltrer passe dans un circuit sanguin extracorporel (112) à travers une chambre à sang (110) d'un dialyseur (113) divisé par une membrane semiperméable (111) en la chambre à sang (110) et en une chambre à liquide de dialyse (108) et du liquide de dialyse (109) passe dans la chambre à liquide de dialyse (108) du dialyseur (113), comportant une pompe à sang (115) pour le contrôle d'un flux sanguin dans le circuit sanguin extracorporel (112), une pompe à liquide de dialyse (107) disposée dans le circuit de liquide de dialyse (109) en amont du dialyseur (113) pour le contrôle d'un flux de liquide de dialyse dans un afflux vers la chambre à liquide de dialyse, un obturateur (117) disposé dans le circuit de liquide de dialyse en aval du dialyseur pour le contrôle du flux de liquide de dialyse dans un reflux hors de la chambre à liquide de dialyse (108),
un dispositif d'équilibrage (104) pour établir un équilibre de liquide dans le circuit de liquide de dialyse entre l'afflux (106) et le reflux (105) de la chambre à liquide de dialyse (108) en tant qu'indice de l'ultrafiltration, et une unité de réglage (101), **caractérisé en ce que** l'unité de réglage est conçue pour régler l'obturateur (117) de manière à obtenir une ultrafiltration prédéfinie.

**2.** Dispositif selon la revendication 1, dans lequel l'unité de réglage (101) est en outre apte à régler la pompe à sang (115) et la pompe à liquide de dialyse (107) de manière à obtenir une ultrafiltration prédéfinie.

**3.** Dispositif (1, 2) de régulation d'une ultrafiltration dans un traitement par dialyse, dans lequel le sang à ultrafiltrer passe dans un circuit sanguin extracorporel (112) à travers une chambre à sang (110) d'un dialyseur (113) divisé par une membrane semiperméable (111) en la chambre à sang (110) et en une chambre à liquide de dialyse (108) et du liquide de dialyse (109) passe dans la chambre à liquide de dialyse (108) du dialyseur (113), comportant une pompe à sang (115) pour le contrôle d'un flux sanguin dans le circuit sanguin extracorporel (112), une pompe à liquide de dialyse disposée dans le circuit de liquide de dialyse (109) en aval du dialyseur (113) pour le contrôle d'un flux de liquide de dialyse dans un reflux hors de la chambre à liquide de dialyse,
un obturateur disposé dans le circuit de liquide de dialyse en amont du dialyseur pour le contrôle du flux de liquide de dialyse dans un afflux vers la chambre à liquide de dialyse,
un dispositif d'équilibrage (104) pour établir un équilibre de liquide dans le circuit de liquide de dialyse entre l'afflux (106) et le reflux (105) de la chambre à liquide de dialyse (113) en tant qu'indice de l'ultrafiltration, et une unité de réglage (101), **caractérisé en ce que** l'unité de réglage est conçue pour régler l'obturateur de manière à obtenir une ultrafiltration prédéfinie.

**4.** Dispositif selon la revendication 3, dans lequel l'unité de réglage (101) est en outre apte à régler la pompe à sang (115) et la pompe à liquide de dialyse de manière à obtenir une ultrafiltration prédéfinie.

**5.** Dispositif (1, 2) selon une des revendications 2 ou 4, dans lequel une valeur prédéfinie réglable pour le flux sanguin et l'unité de réglage est apte à régler le flux de liquide de dialyse dans l'afflux et/ou dans le reflux de la chambre à liquide de dialyse.

**6.** Dispositif (1, 2) selon une des revendications 2 ou 4, dans lequel une valeur prédéfinie est réglable pour le flux et l'unité de réglage est apte à réguler le flux sanguin.

**7.** Dispositif (2) selon une des revendications précédentes, dans lequel le dispositif d'équilibrage présente une unité de mesure de flux différentiel (104) pour mesurer le flux différentiel entre un écoulement dans l'afflux vers la chambre à liquide de dialyse (108) et le reflux hors de la chambre à liquide de dialyse, une bifurcation depuis l'afflux ou le reflux pour dévier du liquide de dialyse de l'afflux ou du reflux dans une autre voie d'écoulement (212), de même qu'un dispositif de réglage de la quantité d'écoulement (211) dans l'afflux, dans le reflux et/ou dans votre voie d'écoulement, lequel dispositif de réglage peut être commandé de manière à ce que le flux différentiel mesuré réponde à une condition prédéfinie, et comportant un dispositif (211) de détermination de la quantité d'écoulement dans l'autre voie d'écoulement en tant qu'indice de l'équilibre de liquide.

**8.** Dispositif (1, 2) selon une des revendications 1 à 6, dans lequel un profil de vitesse d'ultrafiltration peut être prédéfini par unité de réglage (101), dans lequel profil alternent des intervalles à vitesse d'ultrafiltration positive et des intervalles à la vitesse d'ultrafiltration négative.

9. Dispositif (2) selon la revendication 7 et la revendication 8, dans lequel la condition prédéfinie du flux différentiel repose sur une intégration du flux différentiel sur un intervalle d'intégration prédéfini.

10. Dispositif (1, 2, 51) selon une des revendications précédentes, dans lequel l'unité de réglage (101) est apte à prédéfinir une vitesse d'ultrafiltration/un volume d'ultrafiltration ou volume d'hémofiltration à soutirer pendant un cycle de traitement.

11. Dispositif (1, 2, 51) selon une des revendications précédentes, dans lequel la pompe à sang est disposée dans une conduite d'alimentation de la chambre à sang.

12. Dispositif (1, 2, 51) selon une des revendications précédentes, dans lequel l'obturateur (117) se présente sous forme d'une soupape cadencée.

Fig. 1

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005044339 A
- WO 9522743 A